# EUROPEAN PATENT SPECIFICATION

(11) **EP 4 205 575 B1**
(45) Date of publication and mention of the grant of the patent: **14.10.2026**
(21) Application number: 22215817.2
(22) Date of filing: 22.12.2022
(51) Int. Cl.: A24F 40/40, A24F 40/485, A24F 40/10

(54) **VAPORIZATION ASSEMBLY AND ELECTRONIC VAPORIZATION DEVICE**
VERDAMPFUNGSANORDNUNG UND ELEKTRONISCHE VERDAMPFUNGSVORRICHTUNG
ENSEMBLE DE VAPORISATION ET DISPOSITIF DE VAPORISATION ÉLECTRONIQUE

(30) Priority: 30.12.2021 CN 202123418258 U
(43) Date of publication of application: 05.07.2023
(73) Proprietor: Jiangmen Moore Technology., Ltd, Jiangmen City, Guangdong 529040 (CN)
(72) Inventor: HUANG, Yongrui, Jiangmen, 529040 (CN)
(74) Representative: Ing. C. Corradini & C. S.R.L.

(56) References cited:
- US-A1- 2016 213 065
- US-A1- 2021 084 976

## Description

### TECHNICAL FIELD

The present application relates to the field of vaporization device technologies, and in particular, to a vaporization assembly and an electronic vaporization device.

### BACKGROUND

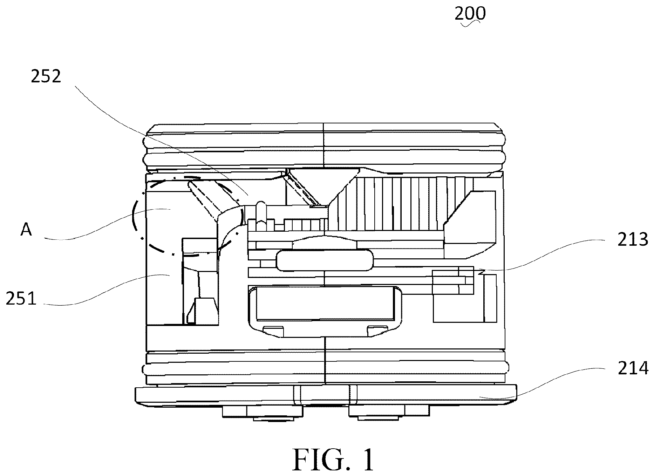

An electronic vaporization device generates an aerosol by vaporizing an aerosol-forming substrate, and a user may obtain effective substances in the aerosol-forming substrate by inhaling the aerosol. When using the existing electronic vaporization device, the user generates a inhaling force, for example, by the mouth, so that the aerosol is transmitted through the airflow channel and collected into a central air outlet channel, and then, is inhaled into the user's mouth. However, in a process of transmitting the aerosol in the airflow channel, the aerosol is prone to be scattered and to form a vortex in a portion A shown in a chain dotted circle in FIG. 1. As a result, the aerosol cannot be completely inhaled into the user's mouth, and thus, the aerosol remaining on the airflow channel may form condensation. The accumulated condensation may be inhaled into the user's mouth, resulting in so-called "leakage phenomenon", which negatively affects the user experience.

Documents US 2016/213065 A1 and US 2021/084976 A1 disclose examples of these existing electronic vaporization devices. In particular, US2016/213065A1 discloses an electronic vaporization device according to the preamble of the independent claim 1.

### SUMMARY

Based on this, it is necessary to provide a vaporization assembly and an electronic vaporization device for the problem of poor user experience of the existing electronic vaporization device.

According to one aspect of the present application, a vaporization assembly is provided, the vaporization assembly includes:
a vaporization base including a mounting cavity and an air outlet;
a vaporization core arranged in the mounting cavity, and cooperating with the vaporization base to define and form a vaporization cavity;
a first airflow channel arranged in the vaporization base in a first direction, an air inlet end of the first airflow channel being in communication with the vaporization cavity; and
a second airflow channel arranged in the vaporization base in a second direction intersecting with the first direction, an air outlet end of the second airflow channel being in communication with the air outlet, and an air inlet end of the second airflow channel being in communication with an air outlet end of the first airflow channel.

According to the invention, a corner between the air inlet end of the second airflow channel and the air outlet end of the first airflow channel is a smooth transition corner.

In an embodiment, a smooth rounded corner is formed between a central axis of the first airflow channel and a central axis of the second airflow channel.

In an embodiment, the vaporization assembly further includes a capillary liquid absorbing structure arranged on the vaporization base and in communication with the second airflow channel. When an inhaling action occurs at the air outlet, an accumulated liquid in the second airflow channel flows into the capillary liquid absorbing structure and is collected by the capillary liquid absorbing structure.

In an embodiment, the capillary liquid absorbing structure includes at least one first capillary groove, and each of first capillary grooves is arranged and spaced apart on the vaporization base in the first direction.

In an embodiment, the capillary liquid absorbing structure includes at least one second capillary groove, and each of the second capillary grooves is arranged and spaced apart on the vaporization base in the second direction.

In an embodiment, the vaporization assembly further includes a liquid guide structure arranged on the vaporization base, and the accumulated liquid in the second airflow channel is guided by the liquid guide structure to flow into the capillary liquid absorbing structure.

In an embodiment, the liquid guide structure includes at least one liquid guide groove, a liquid inlet end of the liquid guide structure is in communication with the second airflow channel, and a liquid outlet end of the liquid guide groove is in communication with the capillary liquid absorbing structure.

According to the invention, the vaporization assembly further includes a vaporization housing including an accommodating cavity, and the vaporization base and the vaporization core are accommodated in the accommodating cavity. An air outlet channel is further provided in the vaporization housing, and the air outlet channel is in communication with the outside and the air outlet.

According to the invention, the vaporization base includes a vaporization top base and a vaporization bottom base, the vaporization top base and the vaporization bottom base cooperate together to form the mounting cavity. The vaporization core is arranged in the mounting cavity, and the vaporization core cooperates with a cavity wall of the mounting cavity to define and form the vaporization cavity. According to the inventio, the air outlet is provided on the vaporization top base.

In an embodiment, the vaporization assembly further includes:
a first seal portion sealedly connected between an outer side wall of the vaporization top base and an inner side wall of the vaporization housing; and
a second seal portion, sealedly connected between an outer side wall of the vaporization bottom
base and the inner side wall of the vaporization housing.

According to another aspect of the present application, an electronic vaporization device is further provided, and the electronic vaporization device includes a vaporization assembly of any one of the above embodiments and a power supply assembly electrically connected to the vaporization assembly.

In the vaporization assembly and electronic vaporization device provided by the embodiments of the present application, in an inhaling process, that is, in a process in which the aerosol flows from the vaporization cavity to the air outlet, the aerosol can flow and turn smoothly at a communication portion between the air outlet end of the first airflow channel and the air inlet end of the second airflow channel, thereby preventing the aerosol from forming an vortex or leakage due to sharp turning, and improving the user's inhaling experience.

### BRIEF DESCRIPTION OF THE DRAWINGS

FIG. 1 is a schematic structural diagram of a partial area of a vaporization assembly in the related art;
FIG. 2 is a schematic structural diagram of a vaporization assembly according to an embodiment of the present application;
FIG. 3 is a schematic diagram illustrating a cross-sectional structure of the vaporization assembly shown in FIG. 2;
FIG. 4 is a schematic diagram illustrating an exploded structure of the vaporization assembly shown in FIG. 2; and
FIG. 5 is a schematic structural diagram illustrating a partial area of the vaporization assembly shown in FIG. 2.
200. Vaporization assembly; 20. vaporization housing; 201. accommodating cavity; 202. air outlet channel; 203. liquid storage cavity; 21. vaporization base; 211. mounting cavity; 212. air outlet; 213. vaporization top base; 214. vaporization bottom base; 215. vaporization cavity; 216. liquid flowing channel; 22. vaporization core; 241. first seal portion; 242. second seal portion; 251. first airflow channel; 252. second airflow channel; 27. capillary liquid absorbing structure; 271. first capillary groove; and 272. second capillary groove.

### DETAILED DESCRIPTION

To make the foregoing objects, features and advantages of the present application more comprehensible, detailed description is made to specific implementations of the present application below with reference to the accompanying drawings. In the following description, many specific details are provided to facilitate a full understanding of the present application. However, the present application may alternatively be implemented in other manners different from those described herein.

Therefore, the present application is not limited to the embodiments disclosed below.

In the description of the present application, it should be understood that orientation or position relationships indicated by the terms such as "center", "longitudinal", "transverse", "length", "width", "thickness", "on", "below", "front", "back", "left", "right", "vertical", "horizontal", "top", "bottom", "inside", "outside", "clockwise", "anticlockwise", "axial", "radial", and "circumferential" are based on orientation or position relationships shown in the accompanying drawings, and are used only for ease and brevity of illustration and description, rather than indicating or implying that the mentioned apparatus or component needs to have a particular orientation or needs to be constructed and operated in a particular orientation. Therefore, such terms should not be construed as limiting of the present application.

In addition, the terms "first" and "second" are used merely for the purpose of description, and shall not be construed as indicating or implying relative importance or implying a quantity of indicated technical features. Therefore, features defining "first" and "second" can explicitly or implicitly include at least one of the features. In description of the present application, "multiple" means at least two, such as two and three, unless it is specifically defined otherwise.

In the present application, it should be noted that unless otherwise clearly specified and limited, the terms "mounted", "connected", "coupled", and "fixed" should be understood in a broad sense, which may, for example, refer to a fixed connection, a detachable connection, a integration; or for example, refer to a mechanical connection or an electrical connection; or for example, refer to a direct connection or an indirect connection through an intermediate medium; or for example, refer to an internal communication between two elements or an interaction relationship between two elements, unless otherwise clearly limited. A person of ordinary skill in the art may understand the specific meanings of the foregoing terms in the present application according to specific situations. In the present application, unless otherwise explicitly specified and defined, a first feature is "on" or "below" a second feature may mean that the first feature and the second feature are in direct, or the first feature and the second feature are in indirect contact through an intermediate medium. In addition, that the first feature is "above", "over", or "on" the second feature may indicate that the first feature is directly above or obliquely above the second feature, or may merely indicate that the horizontal position of the first feature is higher than that of the second feature. That the first feature is "below", "under", and "beneath" the second feature may be that the first feature is directly below or obliquely below the second feature, or may merely indicate that the horizontal position of the first feature is lower than that of the second feature.

It should be noted that, when an element is referred to as "being fixed to" or "being arranged on" another element, the element may be directly on another element, or an intermediate element may be present. When an element is considered to be "connected to" another element, the element may be directly connected to another element, or an intermediate element may also be present. The terms "vertical", "horizontal", "upper", "lower", "left", "right", and similar expressions used in this specification are only for purposes of illustration but not indicate a unique implementation.

The vaporization assembly and the electronic vaporization device of the present application are described below with reference to the accompanying drawings.

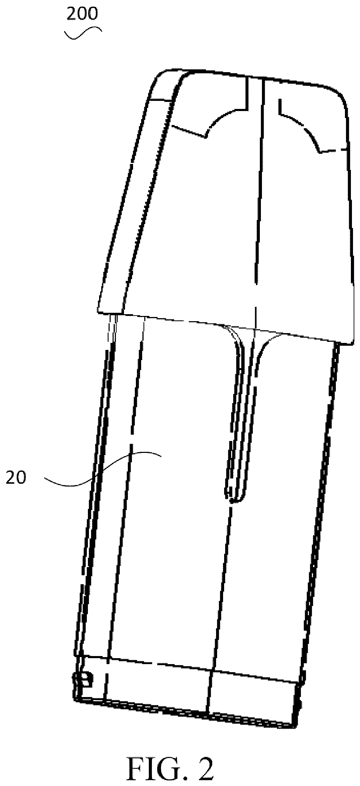

Referring to FIG. 2, an electronic vaporization device according to an embodiment of the present application includes a vaporization assembly 200 and a power supply assembly (not shown in figures) electrically connected to the vaporization assembly 200. The vaporization assembly 200 is configured to store a liquid aerosol-generation substrate and vaporize the aerosol-generation substrate to form an aerosol that can be inhaled by a user. The liquid aerosol-generation substrate may be a liquid substrate such as liquid medicinal, plant grass liquid, or the like. The vaporization assembly 200 can be used in different fields, such as medical treatment, electronic aerosolization, or the like. A power source assembly includes components such as a battery, and the battery is configured to supply power to the vaporization assembly 200, so that the vaporization assembly 200 can vaporize the aerosol-generation substrate to form the aerosol. The vaporization assembly 200 and the power source assembly may be integrated, or may be detachably connected, and may be designed according to specific needs.

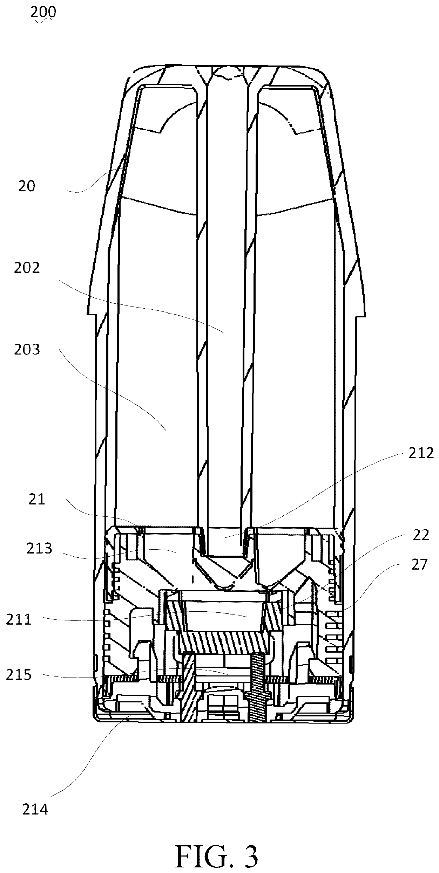

Referring to FIG. 3, the vaporization assembly 200 includes a vaporization housing 20. Specifically, the vaporization housing 20 includes a liquid storage cavity 203 and an air outlet channel 202. The liquid storage cavity 203 is arranged around the air outlet channel 202, and the liquid storage cavity 203 is configured to store the aerosol-generation substrate.

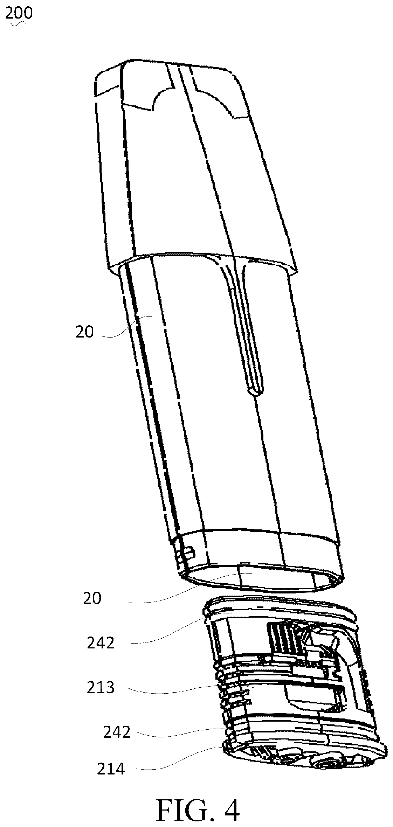

Referring to FIG. 4, the vaporization housing 20 includes an accommodating cavity 201, and the vaporization assembly 200 further includes a vaporization base 21 and a vaporization core 22 that are accommodated in the accommodating cavity 201. The vaporization base 21 includes a vaporization top base 213 and a vaporization bottom base 214. The vaporization bottom base 214 is arranged on a side of the vaporization top base 213 that is away from the liquid storage cavity 203, and cooperates with the vaporization top base 213 to form a mounting cavity 211. The vaporization core 22 is arranged in the mounting cavity 211, and the vaporization core 22 cooperates with a cavity wall of the mounting cavity 211 to define and form a vaporization cavity 215.

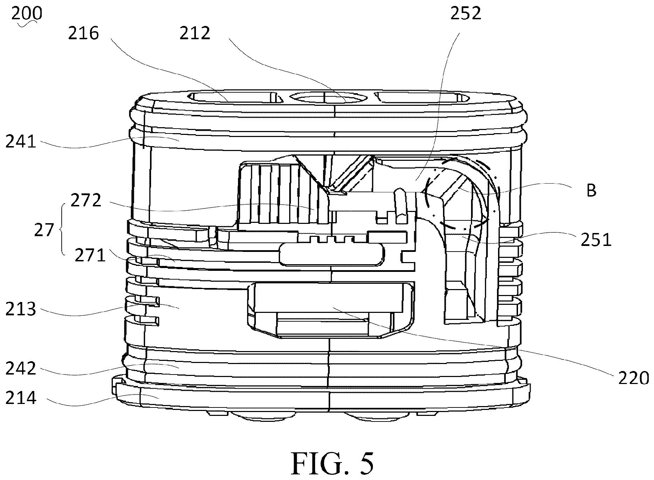

Referring to FIG. 5, the vaporization top base 213 is provided with an air outlet 212 and two liquid flowing channels 216. The two liquid flowing channels 216 are symmetrically arranged on two sides of the air outlet 212 and are in communication between the liquid storage cavity 203 and the vaporization cavity 215. In this way, the aerosol-generation substrate in the liquid storage cavity 203 can enter the vaporization cavity 215 through the two liquid flowing channels 216 to be adsorbed and vaporized by the vaporization core 22. The air outlet channel 202 is in communication with the outside and the air outlet 212, and the air outlet 212 is in communication with the vaporization cavity 215.

In some embodiments, the vaporization assembly 200 further includes a first seal portion 241 sealingly connected between an outer side wall of the vaporization top base 213 and an inner side wall of the vaporization housing 20. Specifically, the first seal portion 241 may be made of a silicone material, and the first seal portion 241 is configured to seal the liquid storage cavity 203 to prevent leakage of the aerosol-generation substrate in the liquid storage cavity 203.

In some embodiments, the vaporization assembly 200 further includes a second seal portion 242, and the second seal portion 242 is sealingly connected between an outer side wall of the vaporization bottom base 214 and an inner side wall of the vaporization housing 20. Specifically, the second seal portion 242 can be made of a silicone material, and the second seal portion 242 is configured to further ensure the sealing performance of the vaporization assembly 200 to the aerosol and the aerosol-generation substrate.

The vaporization core 22 includes a porous liquid guide member and a heating element. The heating element is arranged on a surface of the porous liquid guide member. The porous liquid guide member guides the aerosol-generation substrate to the heating element by using the capillary force, and then, the aerosol-generation substrate is heated and vaporized by the heating element to generate the aerosol. Further, a first airflow channel 251 and a second airflow channel 252 are provided in the vaporization base 21 and are in communication between the vaporization cavity 215 and the air outlet 212.

Referring to FIG. 5, the first airflow channel 251 arranged in the vaporization base 21 in the first direction, and the air inlet end of the first airflow channel is in communication with the vaporization cavity 215. Specifically, the first airflow channel 251 is arranged between the vaporization top base 213 and the vaporization bottom base 214 in the first direction. The first direction may be a length direction of the vaporization base 21. Alternatively, the first direction may be other directions, which are not specifically limited in the present application. The second airflow channel 252 is arranged in the vaporization base 21 in the second direction, and the air outlet end of the second airflow channel 252 is in communication with the air outlet 212. Specifically, the second airflow channel 252 is arranged on the vaporization top base 213 in the second direction. The second direction may be any direction intersecting with the first direction, which is not specifically limited herein in the present application. For example, the second direction may be a width direction of the vaporization base 21. The air inlet end of the second airflow channel 252 is in communication with the air outlet end of the first airflow channel 251. During use, the aerosol generated by heating in the vaporization cavity 215 enters the air outlet channel 202 through the vaporization cavity 215, the first airflow channel 251, the second airflow channel 252, and the air outlet 212 in sequence, and is finally inhaled by the user.

Further, a corner between the air inlet end of the second airflow channel 252 and the air outlet end of the first airflow channel 251 is a smooth transition corner, as shown in the chain dotted circle B in FIG. 5. Specifically, there is a smooth transition between a channel wall of the air inlet end of the second airflow channel 252 and a channel wall of the air outlet end of the first airflow channel 251. In an inhaling process, that is, in a process in which the aerosol flows from the vaporization cavity 215 to the air outlet 212, the aerosol can flow and turn smoothly at a communication portion between the air outlet end of the first airflow channel 251 and the air inlet end of the second airflow channel 252, thereby preventing the aerosol from being scattered to form the vortex or being accumulated to form leakage, due to sharp turning, and improving the inhaling experience of the user.

In some embodiments, a smooth rounded corner is formed between a central axis of the first airflow channel 251 and a central axis of the second airflow channel 252. Specifically, a smooth arc segment is formed at a communication portion between the air outlet end of the first airflow channel 251 and the air inlet end of the second airflow channel 252. In this way, the aerosol is smoothly turned to the air outlet 212 along the arc segment, the accumulation of airflow and the formation of vortex due to the sharp turning are avoided, and thus, the residue and leakage of the aerosol are avoided, thereby improving the inhaling experience of the user.

Referring to FIG. 3 and FIG. 5, in order to more effectively prevent the leakage phenomenon, in an embodiment, the vaporization assembly 200 further includes a capillary liquid absorbing structure 27. The capillary liquid absorbing structure 27 is arranged on the vaporization base 21 and is in communication with the second airflow channel 252. The accumulated liquid in the second airflow channel 252 can flow into the capillary absorbing structure 27 under the action of gravity and/or capillary absorbing force, and be collected by the capillary absorbing structure 27. Specifically, in some embodiments, the channel wall of the second airflow channel 252 that is away from the liquid storage cavity 203 is provided with a liquid accumulation notch, and the second airflow channel 252 is in communication with the capillary liquid absorbing structure 27 through the liquid accumulation notch. During use, under the action of the inhaling force, the accumulated liquid in the second airflow channel 252 can enter and be collected in the capillary liquid absorbing structure 27 from the liquid accumulation notch, thereby reducing the accumulated liquid in the second airflow channel 252. Therefore, it is conducive to alleviate the leakage and improve the user's experience.

In some embodiments, the capillary liquid absorbing structure 27 includes at least one first capillary groove 271, and each of the first capillary grooves 271 is arranged and spaced apart on the vaporization base 21 in the first direction. Specifically, each of the first capillary grooves 271 may be formed by digging out a part of the outer side wall of the vaporization base 21 at intervals in the first direction, and is configured to extend in the second direction. In practical application, each first capillary groove 271 can absorb and store the accumulated liquid through the capillary force, so as to reduce the accumulated liquid in the second airflow channel 252 and improve the user's experience.

In some embodiments, the capillary liquid absorbing structure 27 includes at least one second capillary groove 272, and each of the second capillary grooves 272 is arranged and spaced apart on the vaporization base 21 in the second direction. Specifically, each of the second capillary grooves 272 may be formed by digging out a part of the outer side wall of the vaporization base 21 at intervals in the second direction, and is configured to extend in the first direction. In practical application, each second capillary groove 272 can absorb and store the accumulated liquid through the capillary force, so as to reduce the accumulated liquid in the second airflow channel 252 and improve the user's experience.

It can be understood that the above capillary liquid absorbing structure 27 is exemplary and should not be understood as a limit of the present application.

It should be noted that, in order to further concentrate the aerosol, in some embodiments, the first airflow channel 251 may be designed to be narrower, so that a flowing cross-sectional area of the first airflow channel 251 is reduced, thereby improving the inhaling experience of the user. Specifically, two opposite sides of the vaporization base 21 are defined as a first side and a second side. When the first airflow channel 251 is designed to be narrower, a channel wall of the first airflow channel 251 that is close to the first side can be arranged closer to a channel wall of the second side, so that the first airflow channel 251 can be narrowed.

Referring to FIG. 1 and FIG. 5, in the case of the narrowed first airflow channel 251, the vaporization base 21 includes an additional space, and a volume of the additional space is a volume occupied by the first airflow channel 251 before being narrowed minus a volume occupied by the narrowed first airflow channel 251. Further, the capillary liquid absorbing structure 27 can be arranged in the additional space, so that the aerosol can be more concentrated, the space can be fully utilized, and the liquid leakage prevention effect can be improved.

Referring to FIG. 5, in some embodiments, in order to facilitate the accumulated liquid in the second airflow channel 252 to enter the capillary liquid absorbing structure 27, a liquid guide structure is further provided. The liquid guide structure is arranged on the vaporization base 21, and the accumulated liquid in the second airflow channel 252 is guided by the liquid guide structure to flow into the capillary liquid absorbing structure 27. Specifically, in some embodiments, the liquid guide structure includes a plurality of liquid guide grooves arranged and spaced apart on the vaporization base 21 in the second direction. A liquid inlet end of the liquid guide groove is in communication with the second airflow channel 252, and a liquid outlet end of the liquid guide groove is in communication with the capillary liquid absorbing structure 27. During use, when an inhaling action occurs in the air outlet channel 202, the accumulated liquid in the second airflow channel 252 can enter the liquid guide groove from the liquid inlet end of each liquid guide groove, be guided into the capillary liquid absorbing structure 27, and then flow out from the liquid outlet end of each liquid guide groove.

The technical features in the above embodiments may be randomly combined. For concise description, not all possible combinations of the technical features in the embodiment are described. However, provided that combinations of the technical features do not conflict with each other, the combinations of the technical features are considered as falling within the scope recorded in this specification.

The foregoing embodiments merely express several implementations of the present application.

The descriptions thereof are relatively specific and detailed, but should not be understood as limitations.

## Claims

1. A vaporization assembly (200) including:
a vaporization base (21) including a mounting cavity (211) and an air outlet (212);
a vaporization core (22) arranged in the mounting cavity (211), and cooperating with the vaporization base (21) to define and form a vaporization cavity (216);
a first airflow channel (251) arranged in the vaporization base (21) in a first direction, an air inlet end of the first airflow channel (251) being in communication with the vaporization cavity (216); and
a second airflow channel (252) arranged in the vaporization base (21) in a second direction intersecting with the first direction, an air outlet end of the second airflow channel (252) being in communication with the air outlet (212), and an air inlet end of the second airflow channel (252) being in communication with an air outlet end of the first airflow channel (251),
wherein a corner between the air inlet end of the second airflow channel (252) and the air outlet end of the first airflow channel (251) is a smooth transition corner;
a vaporization housing (20) including an accommodating cavity (201),
wherein the vaporization base (21) and the vaporization core (22) are accommodated in the accommodating cavity (201), an air outlet channel (202) is further provided in the vaporization housing (20), and the air outlet channel (202) is in communication with the outside and the air outlet (212),
wherein the vaporization base (21) includes a vaporization top base (213) and a vaporization bottom base (214), the vaporization core (22) is arranged in the mounting cavity (211), and the vaporization core (22) cooperates with a cavity wall of the mounting cavity (211) to define and form the vaporization cavity (216),
**characterized in that** the vaporization top base (213) and the vaporization bottom base (214) cooperate together to form the mounting cavity (211), wherein the air outlet (212) is provided on the vaporization top base (213).

2. The vaporization assembly (200) according to claim 1, wherein a smooth rounded corner is formed between a central axis of the first airflow channel (251) and a central axis of the second airflow channel (252).

3. The vaporization assembly (200) according to claim 1, further including a capillary liquid absorbing structure (27) arranged on the vaporization base (21) and in communication with the second airflow channel (252),
wherein an accumulated liquid in the second airflow channel (252) flows into the capillary liquid absorbing structure (27) and is collected by the capillary liquid absorbing structure (27) when an inhaling action occurs at the air outlet (212).

4. The vaporization assembly (200) according to claim 3, wherein the capillary liquid absorbing structure (27) includes at least one first capillary groove (271), and each first capillary groove (271) is arranged and spaced apart on the vaporization base (21) in the first direction.

5. The vaporization assembly (200) according to claim 3, the capillary liquid absorbing structure (27) includes at least one second capillary groove (272), and each second capillary groove(272) is arranged and spaced apart on the vaporization base (21) in the second direction.

6. The vaporization assembly (200) according to claim 3, further including a liquid guide structure arranged on the vaporization base (21),
wherein the accumulated liquid in the second airflow channel (252) is guided by the liquid guide structure to flow into the capillary liquid absorbing structure (27) through the liquid guide structure.

7. The vaporization assembly (200) according to claim 6, wherein the liquid guide structure includes at least one liquid guide groove, a liquid inlet end of the liquid guide groove is in communication with the second airflow channel (252), and a liquid outlet end of the liquid guide groove is in communication with the capillary liquid absorbing structure (27).

8. The vaporization assembly (200) according to claim 1, further including:
a first seal portion (241) sealedly connected between an outer side wall of the vaporization top base (213) and an inner side wall of the vaporization housing (20); and
a second seal portion (242) sealedly connected between an outer side wall of the vaporization bottom base (214) and the inner side wall of the vaporization housing (20).

9. An electronic vaporization device, **characterized by** including:
a vaporization assembly (200) according to any one of claims 1 to 8; and
a power supply assembly electrically connected to the vaporization assembly (200).

## Patentansprüche

1. Verdampfungsbaugruppe (200), die Folgendes beinhaltet:
eine Verdampfungsbasis (21), die einen Einbauhohlraum (211) und einen Luftauslass (212) beinhaltet;
einen Verdampfungskern (22), der in dem Einbauhohlraum (211) angeordnet ist und mit der Verdampfungsbasis (21) zusammenwirkt, um einen Verdampfungshohlraum (216) zu definieren und zu bilden;
einen ersten Luftströmungskanal (251), der in der Verdampfungsbasis (21) in einer ersten Richtung angeordnet ist, wobei ein Lufteinlassende des ersten Luftströmungskanals (251) mit dem Verdampfungshohlraum (216) in Kommunikation steht, und
einen zweiten Luftströmungskanal (252), der in der Verdampfungsbasis (21) in einer zweiten Richtung angeordnet ist, die die erste Richtung schneidet, wobei ein Luftauslassende des zweiten Luftströmungskanals (252) mit dem Luftauslass (212) in Kommunikation steht und ein Lufteinlassende des zweiten Luftströmungskanals (252) mit einem Luftauslassende des ersten Luftströmungskanals (251) in Kommunikation steht,
wobei eine Ecke zwischen dem Lufteinlassende des zweiten Luftströmungskanals (252) und dem Luftauslassende des ersten Luftströmungskanals (251) eine sanft übergehende Ecke ist;
ein Verdampfungsgehäuse (20), das einen Aufnahmehohlraum (201) beinhaltet,
wobei die Verdampfungsbasis (21) und der Verdampfungskern (22) in dem Aufnahmehohlraum (201) aufgenommen sind, ein Luftauslasskanal (202) ferner in dem Verdampfungsgehäuse (20) vorgesehen ist und der Luftauslasskanal (202) mit der Außenseite und dem Luftauslass (212) in Kommunikation steht,
wobei die Verdampfungsbasis (21) eine oberseitige Verdampfungsbasis (213) und eine unterseitige Verdampfungsbasis (214) beinhaltet, der Verdampfungskern (22) in dem Einbauhohlraum (211) angeordnet ist und der Verdampfungskern (22) mit einer Hohlraumwand des Einbauhohlraums (211) zusammenwirkt, um den Verdampfungshohlraum (216) zu definieren und zu bilden,
**dadurch gekennzeichnet, dass** die oberseitige Verdampfungsbasis (213) und die unterseitige Verdampfungsbasis (214) zusammenwirken, um den Einbauhohlraum (211) zu bilden, wobei der Luftauslass (212) an der oberseitigen Verdampfungsbasis (213) vorgesehen ist.

2. Verdampfungsbaugruppe (200) nach Anspruch 1, wobei zwischen einer Mittelachse des ersten Luftströmungskanals (251) und einer Mittelachse des zweiten Luftströmungskanals (252) eine glatte, abgerundete Ecke ausgebildet ist.

3. Verdampfungsbaugruppe (200) nach Anspruch 1, ferner beinhaltend eine kapillare Flüssigkeitsabsorptionsstruktur (27), die an der Verdampfungsbasis (21) angeordnet ist und mit dem zweiten Luftströmungskanal (252) in Kommunikation steht,
wobei eine in dem zweiten Luftströmungskanal (252) angesammelte Flüssigkeit in die kapillare Flüssigkeitsabsorptionsstruktur (27) strömt und durch die kapillare Flüssigkeitsabsorptionsstruktur (27) aufgefangen wird, wenn an dem Luftauslass (212) ein Inhalationsvorgang stattfindet.

4. Verdampfungsbaugruppe (200) nach Anspruch 3, wobei die kapillare Flüssigkeitsabsorptionsstruktur (27) mindestens eine erste Kapillarnut (271) beinhaltet und jede erste Kapillarnut (271) in der ersten Richtung auf der Verdampfungsbasis (21) angeordnet und beabstandet ist.

5. Verdampfungsbaugruppe (200) nach Anspruch 3, wobei die kapillare Flüssigkeitsabsorptionsstruktur (27) mindestens eine zweite Kapillarnut (272) beinhaltet und jede zweite Kapillarnut (272) in der zweiten Richtung auf der Verdampfungsbasis (21) angeordnet und beabstandet ist.

6. Verdampfungsbaugruppe (200) nach Anspruch 3, die ferner eine auf der Verdampfungsbasis (21) angeordnete Flüssigkeitsleitstruktur beinhaltet,
wobei die in dem zweiten Luftströmungskanal (252) angesammelte Flüssigkeit durch die Flüssigkeitsleitstruktur so geleitet wird, dass sie durch die Flüssigkeitsleitstruktur in die kapillare Flüssigkeitsabsorptionsstruktur (27) strömt.

7. Verdampfungsbaugruppe (200) nach Anspruch 6, wobei die Flüssigkeitsleitstruktur mindestens eine Flüssigkeitsleitnut beinhaltet, ein Flüssigkeitseinlassende der Flüssigkeitsleitnut mit dem zweiten Luftströmungskanal (252) in Kommunikation steht und ein Flüssigkeitsauslassende der Flüssigkeitsleitnut mit der kapillaren Flüssigkeitsabsorptionsstruktur (27) in Kommunikation steht.

8. Verdampfungsbaugruppe (200) nach Anspruch 1, ferner Folgendes beinhaltend:
einen ersten Dichtungsabschnitt (241), der dichtend zwischen einer äußeren Seitenwand der oberseitigen Verdampfungsbasis (213) und einer inneren Seitenwand des Verdampfungsgehäuses (20) verbunden ist, und
einen zweiten Dichtungsabschnitt (242), der dichtend zwischen einer äußeren Seitenwand der unterseitigen Verdampfungsbasis (214) und der inneren Seitenwand des Verdampfungsgehäuses (20) verbunden ist.

9. Elektronische Verdampfungsvorrichtung, **dadurch gekennzeichnet, dass** sie Folgendes beinhaltet:
eine Verdampfungsbaugruppe (200) nach einem der Ansprüche 1 bis 8 und
eine Stromversorgungsbaugruppe, die elektrisch mit der Verdampfungsbaugruppe (200) verbunden ist.

## Revendications

1. Ensemble de vaporisation (200) comprenant :
une base de vaporisation (21) comportant une cavité de montage (211) et une sortie d'air (212) ;
un noyau de vaporisation (22) disposé dans la cavité de montage (211) et coopérant avec la base de vaporisation (21) pour définir et former une cavité de vaporisation (216) ;
un premier canal d'écoulement d'air (251) disposé dans la base de vaporisation (21) dans une première direction, une extrémité d'entrée d'air du premier canal d'écoulement d'air (251) étant en communication avec la cavité de vaporisation (216) ; et
un second canal d'écoulement d'air (252) disposé dans la base de vaporisation (21) dans une seconde direction coupant la première direction, une extrémité de sortie d'air du second canal d'écoulement d'air (252) étant en communication avec la sortie d'air (212), et une extrémité d'entrée d'air du second canal d'écoulement d'air (252) étant en communication avec une extrémité de sortie d'air du premier canal d'écoulement d'air (251),
un coin entre l'extrémité d'entrée d'air du second canal d'écoulement d'air (252) et l'extrémité de sortie d'air du premier canal d'écoulement d'air (251) étant un coin de transition lisse ;
un boîtier de vaporisation (20) comprenant une cavité de logement (201),
la base de vaporisation (21) et le noyau de vaporisation (22) étant logés dans la cavité de logement (201), un canal de sortie d'air (202) étant en outre prévu dans le boîtier de vaporisation (20), et le canal de sortie d'air (202) étant en communication avec l'extérieur et la sortie d'air (212),
la base de vaporisation (21) comprenant une base supérieure (213) de vaporisation et une base inférieure (214) de vaporisation, le noyau de vaporisation (22) étant disposé dans la cavité de montage (211), et le noyau de vaporisation (22) coopérant avec une paroi de cavité de la cavité de montage (211) pour définir et former la cavité de vaporisation (216),
**caractérisé en ce que** la base supérieure (213) de vaporisation et la base inférieure (214) de vaporisation coopèrent ensemble pour former la cavité de montage (211), la sortie d'air (212) étant prévue sur la base supérieure (213) de vaporisation.

2. Ensemble de vaporisation (200) selon la revendication 1, un coin lisse arrondi étant formé entre un axe central du premier canal d'écoulement d'air (251) et un axe central du second canal d'écoulement d'air (252).

3. Ensemble de vaporisation (200) selon la revendication 1, comprenant en outre une structure capillaire (27) d'absorption de liquide disposée sur la base de vaporisation (21) et en communication avec le second canal d'écoulement d'air (252),
un liquide accumulé dans le second canal d'écoulement d'air (252) s'écoulant dans la structure capillaire (27) d'absorption de liquide et étant recueilli par la structure capillaire (27) d'absorption de liquide lorsqu'une action d'inhalation se produit au niveau de la sortie d'air (212).

4. Ensemble de vaporisation (200) selon la revendication 3, la structure capillaire (27) d'absorption de liquide comprenant au moins une première rainure capillaire (271), et chaque première rainure capillaire (271) étant disposée et espacée sur la base de vaporisation (21) dans la première direction.

5. Ensemble de vaporisation (200) selon la revendication 3, la structure capillaire (27) d'absorption de liquide comprenant au moins une seconde rainure capillaire (272), et chaque seconde rainure capillaire (272) étant disposée et espacée sur la base de vaporisation (21) dans la seconde direction.

6. Ensemble de vaporisation (200) selon la revendication 3, comprenant en outre une structure de guidage de liquide disposée sur la base de vaporisation (21),
le liquide accumulé dans le second canal d'écoulement d'air (252) étant guidé par la structure de guidage de liquide pour s'écouler dans la structure capillaire (27) d'absorption de liquide à travers la structure de guidage de liquide.

7. Ensemble de vaporisation (200) selon la revendication 6, la structure de guidage de liquide comprenant au moins une rainure de guidage de liquide, une extrémité d'entrée de liquide de la rainure de guidage de liquide étant en communication avec le second canal d'écoulement d'air (252), et une extrémité de sortie de liquide de la rainure de guidage de liquide étant en communication avec la structure capillaire (27) d'absorption de liquide.

8. Ensemble de vaporisation (200) selon la revendication 1, comprenant en outre :
une première partie d'étanchéité (241) reliée de manière étanche entre une paroi latérale extérieure de la base supérieure (213) de vaporisation et une paroi latérale intérieure du boîtier de vaporisation (20) ; et
une seconde partie d'étanchéité (242) reliée de manière étanche entre une paroi latérale extérieure de la base inférieure (214) de vaporisation et la paroi latérale intérieure du boîtier de vaporisation (20).

9. Dispositif de vaporisation électronique, **caractérisé en ce qu'**il comprend :
un ensemble de vaporisation (200) selon l'une quelconque des revendications 1 à 8 ; et
un ensemble d'alimentation électrique relié électriquement à l'ensemble de vaporisation (200).
